# EUROPÄISCHE PATENTSCHRIFT

(11) **EP 1 280 752 B1**
(45) Veröffentlichungstag und Bekanntmachung des Hinweises auf die Patenterteilung: **28.07.2004**
(21) Anmeldenummer: 01923732.0
(22) Anmeldetag: 23.04.2001
(51) Int. Cl.: C07C 39/28, C07C 37/02, C07C 43/225, C07C 43/23

(54) **VERFAHREN ZUR HERSTELLUNG VON 2-ALKYL-3-CHLORPHENOLEN**
METHOD FOR PRODUCING 2-ALKYL-3-CHLOROPHENOLS
PROCEDE DE PRODUCTION DE 2-ALKYL-3-CHLOROPHENOLS

(30) Priorität: 03.05.2000 DE 10021413
(43) Veröffentlichungstag der Anmeldung: 05.02.2003
(73) Patentinhaber: Bayer CropScience AG, 40789 Monheim (DE)
(72) Erfinder: MÜH, Thorsten, 51375 Leverkusen (DE); WEINTRITT, Holger, 40764 Langenfeld (DE); LANTZSCH, Reinhard, 42115 Wuppertal (DE); HÜBSCH, Walter, 42113 Wuppertal (DE)
(86) Internationale Anmeldenummer: PCT/EP2001/004547
(87) Internationale Veröffentlichungsnummer: WO 2001/083417

(56) Entgegenhaltungen:
- L: TESTAFERRI ET AL: "The reactions of unactivated aryl halides with sodium methoxide in HMPA" TETRAHEDRON., Bd. 39, Nr. 1, 1983, Seiten 193-197, XP002168754 ELSEVIER SCIENCE PUBLISHERS, AMSTERDAM., NL ISSN: 0040-4020
- HOUBEN; WEYL: 'Methoden der organischen Chemie, Band VI/1c', 1976, GEORG THIEME VERLAG, STUTTGART Seiten 158 - 166

## Beschreibung

Die Erfindung betrifft ein neues Verfahren zur Herstellung von 2-Alkyl-3-chlorphenolen.

2-Alkyl-3-chlorphenole sind Zwischenprodukte, die beispielsweise für die Herstellung von Pflanzenschutzmitteln verwendet werden können (vgl. WO 98/21189).

Es ist bereits bekannt geworden (Siehe Tetrahedron, 39 (1), 193-197), dass man 3-Chlor-2-methylphenol durch Umsetzung von 2-Chlortoluol mit Natriummethanolat in Hexamethylenphosphorsäuretriamid (HMPA) als Lösungsmittel und Behandlung der Reaktionslösung mit Natriumisopropylthiolat erhält. Der Nachteil dieses Verfahrens liegt zum einen in der Verwendung von HMPA als Lösungsmittel, da dieses stark krebserregend ist. Außerdem ist das verwendete Natriumisopropylthiolat bzw. das bei der Aufarbeitung freigesetzte Isopropylthiol sehr geruchsintensiv. Aus diesen Gründen ist dieses Verfahren nicht in großtechnischem Maßstab anwendbar.

In einem anderen Verfahren (vgl. Justus Liebigs Ann. Chem., 350, 1906, 112) erfolgt die Darstellung von 3-Chlor-2-methylphenol ausgehend von 2-Amino-6-chlortoluol. Durch Diazotierung mit salpetriger Säure und anschließender Phenolverkochung in einem siedenden Gemisch aus Wasser und Schwefelsäure erhält man 3-Chlor-2-methylphenol.

Ein wesentlicher Nachteil dieses Verfahrens ist, dass das Ausgangsprodukt nicht isomerenrein hergestellt werden kann. Es werden hohe Anteile an Nebenprodukten isoliert, wodurch geringere Ausbeuten erhalten werden. Außerdem ist das gebildete Diazoniumsalz schlecht löslich, so dass das Verfahren in hoher Verdünnung durchgeführt werden muss, wodurch die Herstellung von 3-Chlor-2-methylphenol im großtechnischen Maßstab erschwert wird. Ein weiterer Nachteil ist, dass eine Wasserdampfdestillation durchgeführt werden muss, deren Durchführung vor allem im großtechnischen Maßstab aufwendig ist.

Es wurde nun gefunden, dass man 2-Alkyl-3-chlorphenole der allgemeinen Formel (I) 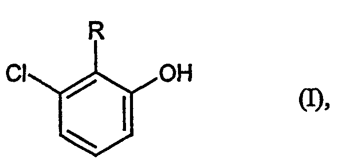 in welcher
- R: für C₁-C₆-Alkyl steht,
erhält, wenn man Alkyldichlorbenzol-Derivate der allgemeinen Formel (II) 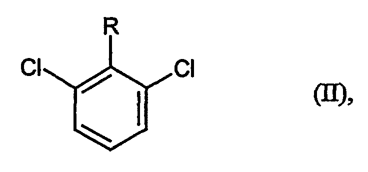 in welcher
- R: die oben genannten Bedeutungen hat,

a1) mit einer Base in einem hochsiedenden organischen Verdünnungsmittel der allgemeinen Formel (III)

   R¹- OH (III),

   in welcher
   - R¹ für: R²-O-(CH₂)₂-O-(CH₂)₂-,
   R²-O-(CH₂)₂-O(-CH₂)₂-O-(CH₂)₂-,
   R²-O-(CH₂)₂-O(-CH₂)₂-O-(CH₂)₂-O-(CH₂)₂- oder
   -C₆ bis C₁₀-Alkyl steht,
   wobei R² für Wasserstoff, Methyl oder Ethyl steht,
   gegebenenfalls in Gegenwart eines Katalysators umsetzt, und das bei der Umsetzung gegebenenfalls freiwerdende Wasser kontinuierlich entfernt,
   oder
a2) mit einer Base in einem hochsiedenden organischen Verdünnungsmittel der allgemeinen Formel (III)

   R¹- OH (III),

   in welcher
   R¹ die oben angegebenen Bedeutungen hat,
   gegebenenfalls in Gegenwart eines Katalysators umsetzt, und das gegebenenfalls bei der Umsetzung freiwerdende Wasser kontinuierlich entfernt,
   und die dabei entstehenden Verbindungen der Formel (IV) 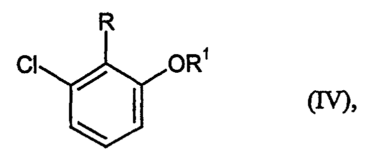 in welcher
   - R und R¹: die oben angegebenen Bedeutungen haben,
   mit höher konzentrierter Säure behandelt,
   oder
b1) mit einer Base in einem primären Alkohol mit 1 bis 3 Kohlenstoffatomen als Verdünnungsmittel unter Druck umsetzt,
   oder
b2) mit einer Base in einem Verdünnungsmittel der allgemeinen Formel (III)

   R¹-OH (III)

   in welcher R¹ die oben angegebenen Bedeutungen hat, unter Druck umsetzt,
   und die dabei entstehenden Verbindungen der Formel (IV) 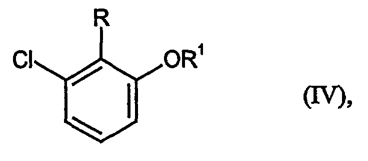 in welcher
   - R und R¹: die oben angegebenen Bedeutungen haben,
   mit höher konzentrierter Säure behandelt.

Im Anschluss an die Verfahrensvarianten a1) und b1) wird das Reaktionsgemisch mit einer verdünnten Säure angesäuert.

In den Verbindungen der Formel (II) steht R insbesondere für Methyl, Ethyl, n- oder i-Propyl.

In den Verbindungen der Formel (II) steht R besonders bevorzugt für Methyl.

In den Verbindungen der Formel (III) steht R¹ besonders bevorzugt für HO-(CH₂)₂-O-(CH₂)₂-.

Die oben aufgeführten oder in Vorzugsbereichen angegebenen Restdefinitionen gelten sowohl für die Ausgangsverbindungen der Formeln (II) und (III) als auch entsprechend für die Endprodukte der Formel (I) und die Zwischenprodukte der Formel (IV).

Die in den jeweiligen Kombinationen bzw. Kombinationen von Resten im einzelnen angegeben Restdefinitionen werden unabhängig von der jeweilig angegebenen Kombination der Reste, beliebig auch durch Restedefinition anderer Vorzugsbereiche ersetzt.

Es ist als ausgesprochen überraschend zu bezeichnen, dass im erfindungsgemäßen Verfahren 2-Alkyl-3-chlorphenole in hohen Ausbeuten und hoher Reinheit erhalten werden, da für vergleichbare Umsetzungen sonst drastischere Reaktionsbedingungen, wie beispielsweise die Phenolverkochung in einem siedenden Gemisch aus Wasser und Schwefelsäure, erforderlich sind.

Das erfindungsgemäße Verfahren weist eine Reihe von Vorteilen auf. So lassen sich 2-Alkyl-3-chlorphenole unter Verwendung nicht krebserregender Lösungsmittel herstellen, ohne dass die Umsetzung in großer Verdünnung durchgeführt werden muss. Daher ist das neue Verfahren, insbesondere für die großtechnische Anwendung gut geeignet.

Die Verbindungen der allgemeinen Formel (IVa) sind noch nicht bekannt und als neue chemische Verbindungen ebenfalls Gegenstand der vorliegenden Anmeldung

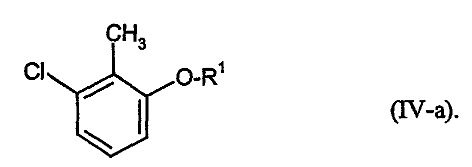

In den Verbindungen der Formel (IVa) steht
- R¹: für R²-O-(CH₂)₂-O-(CH₂)₂-,
R²-O-(CH₂)₂-O(-CH₂)₂-O-(CH₂)₂-, oder
R²-O-(CH₂)₂-O(-CH₂)₂- O-(CH₂)₂- O-(CH₂)₂-,
wobei R² für Wasserstoff, Methyl oder Ethyl steht.

In den Verbindungen der Formel (IVa) steht R¹ besonders bevorzugt für HO-(CH₂)₂-O-(CH₂)₂-.

Die Alkyldichlorbenzol-Derivate der allgemeinen Formel (II) und alle anderen Ausgangsverbindungen sind gängige Handelsprodukte oder können durch einfache Verfahren aus diesen hergestellt werden.

Als Verdünnungsmittel der allgemeinen Formel (III) zur Durchführung der Verfahrensvarianten a1) und a2) kommen beispielhaft und vorzugsweise Diethylenglykol, Triethylenglykol, Tetraethylenglykol oder höher siedende primäre Alkohole in Betracht. Bevorzugt wird in den Verfahrensvarianten a1) und a2) Diethylenglykol verwendet.

Die erfindungsgemäßen Verfahrensvarianten a1) und a2) werden in Gegenwart eines geeigneten Säureakzeptors durchführt. Hierzu gehören beispielhaft und vorzugsweise Erdalkalimetall- oder Alkalimetallhydroxide, -alkoholate oder -carbonate, wie beispielsweise Natriummethanolat, Natriummethanolat, Kalium-tert.-butanolat, Natriumhydroxid, Kaliumhydroxid, Natriumcarbonat oder Kaliumcarbonat. Bevorzugt werden in den Verfahrensvarianten a1) und a2) Natriumhydroxid oder insbesondere Kaliumhydroxid verwendet.

Die erfindungsgemäßen Verfahrensvarianten a1) und a2) werden gegebenenfalls in Gegenwart eines geeigneten Katalysators durchgeführt. Hierzu gehören vorzugsweise tertiäre Amine, wie Trimethylamin, Triethylamin, Tributylamin, N,N-Dimethylanilin, N,N-Dimethyl-benzylamin, Pyridin, Picoline, 2-Methyl-5-ethyl-pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Zur Durchführung der erfindungsgemäßen Verfahrensvariante a1) werden verdünnte Säuren, insbesondere Mineralsäuren, beispielhaft und vorzugsweise Schwefelsäure, Phosphorsäure, insbesondere Salzsäure verwendet.

Die Verfahrensvariante a1) kann als Eintopfverfahren durchgefürt werden.

Zur Durchführung der erfindungsgemäßen Verfahrensvariante a2) werden höher konzentrierte Säuren, insbesondere Mineralsäuren, beispielhaft und vorzugsweise Schwefelsäure oder Salzsäure oder Bromwasserstoff; sowie Lewissäuren, beispielhaft und vorzugsweise Aluminiumtrichlorid, Bortrichlorid oder Bortribromid verwendet. Bevorzugt wird in der Verfahrensvariante a2) Schwefelsäure verwendet.

Die Reaktionstemperaturen können bei der Durchführung des erfindungsgemäßen Verfahrensvariante a1) in einem größeren Bereich variiert werden. Im Allgemeinen arbeitet man bei Temperaturen von 100 bis 250°C, vorzugsweise bei Temperaturen von 160 bis 230°C, besonders bevorzugt bei Temperaturen von 180 bis 220°C.

Die Reaktionstemperaturen können bei der Durchführung des erfindungsgemäßen Verfahrensvariante a2) in einem größeren Bereich variiert werden. Im Allgemeinen arbeitet man bei Temperaturen von 100 bis 250°C, vorzugsweise bei Temperaturen von 120 bis 230°C, besonders bevorzugt bei Temperaturen von 140 bis 200°C.

Die erfindungsgemäßen Verfahrenvarianten a1) und a2) werden im Allgemeinen unter Normaldruck durchgeführt. Es ist jedoch auch möglich, unter Druck - im Allgemeinen zwischen 1 bar und 10 bar zu arbeiten.

Zur Durchführung der erfindungsgemäßen Verfahrensvariante a1) zur Herstellung der Verbindungen der Formel (I) setzt man pro Mol der Verbindungen der Formel (II) im Allgemeinen 2 bis 10 Mol, vorzugsweise 2 bis 4 Mol Base ein.

Zur Durchführung der erfindungsgemäßen Verfahrensvariante a2) zur Herstellung der Verbindungen der Formel (I) setzt man pro Mol der Verbindungen der Formel (II) im Allgemeinen 1 bis 10 Mol, vorzugsweise 1 bis 3 Mol Base ein.

Zur Durchführung der erfindungsgemäßen Verfahrensvariante a1) geht man im Allgemeinen wie folgt vor: Die Alkyldichlorbenzol-Derivate der allgemeinen Formel (II) werden mit einer Base in Gegenwart eines Verdünnungsmittels erhitzt, wobei das bei der Umsetzung freigesetzte Wasser kontinuierlich abdestilliert wird. Zur Aufarbeitung wird das Reaktionsgemisch mit einer Säure angesäuert und in üblicher Weise aufgearbeitet.

Zur Durchführung der erfindungsgemäßen Verfahrensvariante a2) geht man im Allgemeinen wie folgt vor: Die Alkyldichlorbenzol-Derivate der allgemeinen Formel (II) werden mit einer Base in Gegenwart eines Verdünnungsmittels erhitzt, wobei das bei der Umsetzung freigesetzte Wasser kontinuierlich abdestilliert wird, und die Reaktionsmischung in üblicher Weise aufgearbeitet wird. Die dabei erhaltenenen Verbindungen der Formel (IV) werden mit Säure behandelt, das Reaktionsgemisch wird in üblicher Weise aufgearbeitet.

Als Verdünnungsmittel zur Durchführung der erfindungsgemäßen Verfahrensvarianten b1) und b2) kommen primäre Alkohole, wie Methanol, Ethanol, n-Propanol oder i-Propanol, oder deren Gemische mit Wasser in Betracht. Bevorzugt wird in den Verfahrensvarianten b1) und b2) Methanol verwendet.

Die erfindungsgemäßen Verfahrensvarianten b1) und b2) werden in Gegenwart eines geeigneten Säureakzeptors durchführt. Als solche kommen alle üblichen anorganischen oder organischen Basen infrage. Hierzu gehören beispielhaft und vorzugsweise Erdalkalimetall- oder Alkalimetallhydroxide, -alkoholate oder -carbonate, wie beispielsweise Natriummethanolat, Natriummethanolat, Kalium-tert.-butanolat, Natriumhydroxid, Kaliumhydroxid, Natriumcarbonat oder Kaliumcarbonat. Bevorzugt werden in den Verfahrensvarianten b1) und b2) Natriumhydroxid oder insbesondere Kaliumhydroxid verwendet.

Zur Durchführung der erfindungsgemäßen Verfahrensvariante b1) werden verdünnte Säuren, insbesondere Mineralsäuren, beispielhaft und vorzugsweise Schwefelsäure, Phosphorsäure, insbesondere Salzsäure verwendet.

Zur Durchführung der erfindungsgemäßen Verfahrensvariante b2) werden höher konzentrierte Säuren, insbesondere Mineralsäuren, beispielhaft und vorzugsweise Schwefelsäure oder Salzsäure oder Bromwasserstoff; sowie Lewissäuren, beispielhaft und vorzugsweise Aluminiumtrichlorid, Bortrichlorid oder Bortribromid verwendet. Bevorzugt wird in der Verfahrensvariante a2) Schwefelsäure verwendet.

Die Reaktionstemperaturen können bei der Durchführung der erfindungsgemäßen Verfahrensvariante b1) in einem größeren Bereich variiert werden. Im Allgemeinen arbeitet man bei Temperaturen von 170 bis 250°C, vorzugsweise bei Temperaturen von 180 bis 220°C.

Die Reaktionstemperaturen können bei der Durchführung der erfindungsgemäßen Verfahrensvariante b2) in einem größeren Bereich variiert werden. Im Allgemeinen arbeitet man bei Temperaturen von 100 bis 220°C, vorzugsweise bei Temperaturen von 150 bis 200°C.

Die erfindungsgemäßen Verfahrensvarianten b1) und b2) werden im Allgemeinen unter erhöhtem Druck durchgeführt. Im Allgemeinen arbeitet man bei Drücken von 1 bis 130 bar, insbesondere bei Drücken von 10 bis 40 bar.

Zur Durchführung der erfindungsgemäßen Verfahrensvariante b1) zur Herstellung der Verbindungen der Formel (I) setzt man pro Mol der Verbindungen der Formel (II) im Allgemeinen 2 bis 10 Mol, vorzugsweise 2 bis 4 Mol Base ein.

Zur Durchführung der erfindungsgemäßen Verfahrensvariante b2) zur Herstellung der Verbindungen der Formel (I) setzt man pro Mol der Verbindungen der Formel (II) im Allgemeinen 1 bis 10 Mol, vorzugsweise 1 bis 3 Mol Base ein.

Zur Durchführung der erfindungsgemäßen Verfahrensvariante b1) geht man im Allgemeinen wie folgt vor: Die Alkyldichlorbenzol-Derivate der allgemeinen Formel (II) werden mit einer Base in Gegenwart eines Verdünnungsmittels unter Druck erhitzt. Nach beendeter Reaktion wird das Reaktionsgemisch mit einer Säure angesäuert und in üblicher Weise aufgearbeitet.

Zur Durchführung der erfindungsgemäßen Verfahrensvariante b2) geht man im Allgemeinen wie folgt vor: Die Alkyldichlorbenzol-Derivate der allgemeinen Formel (II) werden mit einer Base in Gegenwart eines Verdünnungsmittels unter Druck erhitzt. Die dabei erhaltenenen Verbindungen der Formel (IV) werden mit Säure behandelt, das Reaktionsgemisch wird in üblicher Weise aufgearbeitet.

Die erfindungsgemäße Verfahrensvariante a1) ist besonders bevorzugt.

Das erfindungsgemäße Verfahren wird insbesondere zur Herstellung von 3-Chlor-2-methyl-phenol, das ein wichtiges Zwischenprodukt für die Herstellung von Schädlingsbekämpfungsmitteln ist (vgl. WO 98/21189), verwendet. Nach dem erfindungsgemäßen Verfahren wird 3-Chlor-2-methylphenol in konstant hohen Reinheiten und guten Ausbeuten erhalten. Das neue Verfahren erleichtert daher die Herstellung von bekannten Schädlingsbekämpfungsmitteln

Die nachfolgenden Beispiele dienen zur Erläuterung der Erfindung. Die Erfindung ist jedoch nicht auf die Beispiele limitiert.

### Beispiele

### Beispiel 1

### 3-Chlor-2-methylphenol

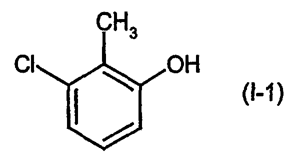

### Verfahrensvariante a1)

16,1 g (0,1 mol) 2,6-Dichlor-toluol und 19,8 g (0,3 mol) Kaliumhydroxid (85 %ig) werden in 30 ml Diethylenglycol 18 Stunden bei einer Badtemperatur von 190°C erhitzt. Dabei wird das bei der Reaktion freigesetzte Wasser abdestilliert. Nach dem Abkühlen wird die Reaktionsmischung mit 100 ml Wasser gerührt, bis eine Lösung erhalten wird und dreimal mit 50 ml Dichlormethan extrahiert. Die Wasserphase wird mit 35 ml 30 %iger Salzsäure versetzt und dreimal mit 70 ml Dichlormethan extrahiert. Die organischen Phasen werden über Natriumsulfat getrocknet und im Vakuum eingeengt. Man erhält 3-Chlor-2-methylphenol als Feststoff (12,4 g, NMR Gehalt: 83 %, 72 % d. Th).

NMR (D-6-DMSO): 6.75 (d, 1H, Aromat), 6.85 (d, 1H, Aromat) 7.0 (t, 1H, Aromat), 9.8 (s, 1H, Phenol-OH)

### Beispiel 2

### 3-Chlor-2-methylphenol

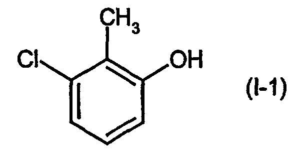

### Verfahrensvariante a1)

16,1 g ( 0,1 mol) 2,6-Dichlortoluol, 19,8 g (0,3 mol) Kaliumhydroxid (85%ig) und 0,3 g Dibenzo-18-Krone-6 werden in 30 ml Diethylenglycol 16 Stunden bei einer Badtemperatur von 190°C unter Rückfluss erhitzt. Dabei wird das bei der Reaktion freigesetzte Wasser abdestilliert. Nach dem Abkühlen wird die Reaktionsmischung mit 100 ml Wasser gerührt, bis eine Lösung erhalten wird und dreimal mit 50 ml Dichlormethan extrahiert. Die Wasserphase wird mit 35 ml 30 %iger Salzsäure versetzt und dreimal mit 70 ml Dichlormethan extrahiert. Die organischen Phasen werden getrocknet und im Vakuum eingeengt. Man erhält 3-Chlor-2-methylphenol als Feststoff (11,5 g, HPLC-Gehalt: 97%, 78% d. Th).

### Beispiel 3

### 3-Chlor-2-methyl-phenol

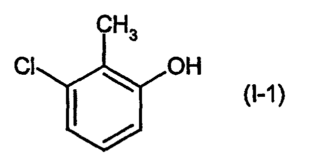

### Verfahrensvariante b1)

8,1 g (0,05 mol) 2,6-Dichlor-toluol und 9,9 g (0,15 mol) Kaliumhydroxid (85 %ig) werden in 40 ml Methanol im Autoklaven 20 Stunden auf 200°C erhitzt, wobei ein Eigendruck von 30 bar entsteht. Nach dem Abkühlen wird die Reaktionsmischung auf 250 ml Wasser gegossen und dreimal mit 70 ml Dichlormethan extrahiert. Die Wasserphase wird mit 30 %iger Salzsäure auf pH 1-2 angesäuert und wiederum dreimal mit je 70 ml Dichlormethan extrahiert. Die vereinigten organischen Phasen werden über Natriumsulfat getrocknet und im Vakuum zu einem Feststoff eingedampft. Man erhält 3-Chlor-2-methylphenol als Feststoff (6,2 g, HPLC-Gehalt: 93,5%, 82% d. Th).

### Beispiel 4

### 3-Chlor-2-methyl-anisol

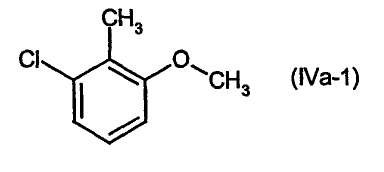

### Verfahrensvariante b2)- erste Stufe - Herstellung von Verbindungen der Formel (IVa-1)

8.1 g (0.05 mol) 2,6-Dichlor-toluol, und 9.9 g (0.15 mol) Kaliumhydroxid (85 %ig) werden in 40 ml Methanol im Autoklaven 20 Stunden auf 160°C erhitzt, wobei der Druck auf ca. 12 bar steigt. Nach dem Abkühlen wird auf ca. 250 ml Wasser gegossen und dreimal mit ca. 70 ml Dichlormethan extrahiert. Die vereinigten org. Phasen werden über Natriumsulfat getrocknet und im Vakuum eingedampft. Man erhält 3-Chlor-2-methyl-anisol als Öl (2,5 g, GC-Analyse: 37,1 %,12 % d. Th).

### Ret. Index GC: 1187, m/e: 156

NMR (D6-DMSO) ppm: 2.2 (s, 3H, CH3), 3.8 s, 3H, O-CH3), 6.95 (d, 1H, Aromat), 7.0 (d, 1H, Aromat), 7.2 (t, 1H, Aromat)

### 3-Chlor-2-methyl-phenol

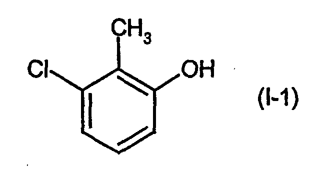

### Verfahrensvariante b2) - zweite Stufe - Herstellung von Verbindungen der Formel (I-1)

3,1 g (0,02 mol) 3-Chlor-2-methyl-anisol werden in 20 ml 50%iger Schwefelsäure 18 Stunden unter Rückfluss erhitzt. Nach dem Abkühlen wird dreimal mit Dichlormethan extrahiert, über Natriumsulfat getrocknet und im Vakuum zur Trockne eingeengt. Man erhält 3-Chlor-2-methylphenol als Feststoff (2,9 g, HPCL-Gehalt: 11, 7, 12 % d. Th).

### Beispiel 5

### 2-Chlor-6-[2(-2-hydroxy-ethoxy)-ethoxy]-toluol

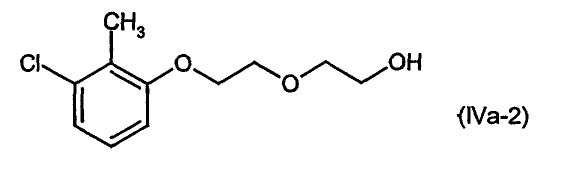

### Verfahrensvariante b2)- erste Stufe - Herstellung von Verbindungen der Formel (IVa-2)

4 g (0.025 mol) 2,6-Dichlor-toluol und 2,4 g (0.036 mol) Kaliumhydroxid (85 %ig) werden in 10 ml Diethylenglycol vier Stunden unter Rückfluss erhitzt. Nach dem Abkühlen wird die Reaktionsmischung mit Wasser kräftig gerührt und dreimal mit Dichlormethan extrahiert. Die organischen Phasen werden über Natriumsulfat getrocknet und im Vakuum zu eingeengt. Man erhält 2-Chlor-6-[2(-2-hxdroxyethoxy)-ethoxy]-toluol als Öl (2,7 g, GC-Analyse: 32,3 %, 15 % d. Th).

### Ret. Index GC: 1776, m/e: 230

NMR (D6-DMSO) ppm: 2.2 (s, 3H, CH3), 3.5 (m, 4H, CH2), 3.8 m, 2H, CH2), (4.1, m, 2H, CH2), 4.6 (m, 1H, OH), 6.9 - 7.2 (m, 3H, Aromaten-H)

## Patentansprüche

1. Verfahren zur Herstellung von Verbindungen der Formel (I) 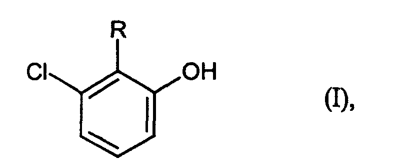 in welcher
R für C₁-C₆-Alkyl steht,
**dadurch gekennzeichnet, dass** man Verbindungen der allgemeinen Formel (II) 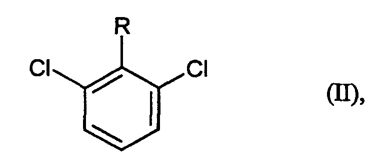 in welcher
R die oben genannten Bedeutungen hat,
a1) mit einer Base in einem hochsiedenden organischen Verdünnungsmittel der allgemeinen Formel (III)
R¹- OH (III),
in welcher
R¹ für R²-O-(CH₂)₂-O-(CH₂)₂-,
R²-O-(CH₂)₂-O(-CH₂)₂-O-(CH₂)₂-,
R²-O-(CH₂)₂-O(-CH₂)₂-O-(CH₂)₂- O-(CH₂)₂- oder
-C₆ bis C₁₀-Alkyl steht,
wobei R² für Wasserstoff, Methyl oder Ethyl steht,
gegebenenfalls in Gegenwart eines Katalysators umsetzt, und das bei der Umsetzung gegebenenfalls freiwerdende Wasser kontinuierlich entfernt,
oder
a2) mit einer Base in einem hochsiedenden organischen Verdünnungsmittel der allgemeinen Formel (III)
R¹- OH (III),
in welcher R¹ die oben angegebenen Bedeutungen hat,
gegebenenfalls in Gegenwart eines Katalysators umsetzt, und das gegebenenfalls bei der Umsetzung freiwerdende Wasser kontinuierlich entfernt,
und die dabei entstehenden Verbindungen der Formel (IV) 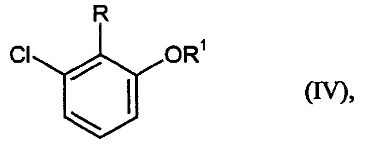 in welcher
R und R¹ die oben angegebenen Bedeutungen haben,
mit höher konzentrierter Säure behandelt,
oder
b1) mit einer Base in einem primären Alkohol mit 1 bis 3 Kohlenstoffatomen als Verdünnungsmittel bei Drücken von 10 bis 40 bar umsetzt,
oder
b2) mit einer Base in einem Verdünnungsmittel der allgemeinen Formel (III)
R¹-OH (III),
in welcher
R¹ die oben angegebenen Bedeutungen hat,
unter Druck umsetzt,
und die dabei entstehenden Verbindungen der Formel (IV) 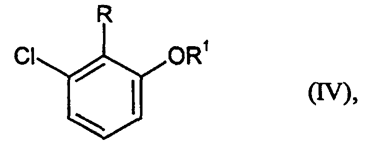 in welcher
R und R¹ die oben angegebenen Bedeutungen haben,
mit höher konzentrierter Säure behandelt.

2. Verfahren gemäß Anspruch 1, **dadurch gekennzeichnet, dass** in den Verfahrensvarianten a1) und a2) Diethylenglykol als Verdünnungsmittel verwendet wird.

3. Verfahren gemäß Anspruch 1, **dadurch gekennzeichnet, dass** in den Verfahrensvarianten b1) und b2) Methanol als Verdünnungsmittel verwendet wird.

4. Verfahren gemäß mindestens einem der Ansprüche 1 bis 2, **dadurch gekennzeichnet, dass** die Verfahrensvarianten a1) und a2) bei Temperaturen von 100°C bis 250°C durchgeführt werden.

5. Verfahren gemäß mindestens einem der Ansprüche 1 oder 3, **dadurch gekennzeichnet, dass** die Verfahrenvariante b1) bei Temperaturen von 170°C bis 250°C durchgeführt wird.

6. Verfahren gemäß mindestens einem der Ansprüche 1 oder 3, **dadurch gekennzeichnet, dass** die Verfahrensvariante b2) bei Temperaturen von 100°C bis 220°C durchgeführt wird.

7. Verfahren gemäß mindestens einem der Ansprüche 1, 2 oder 4, **dadurch gekennzeichnet, dass** die Verfahrensvariante a1) als Eintopfverfahren durchgeführt wird.

8. Verbindungen der Formel (IVa) 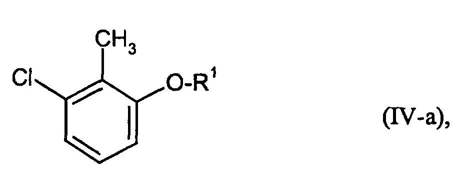 in welcher
R¹ für R²-O-(CH₂)₂-O-(CH₂)₂-,
R²-O-(CH₂)₂-O(-CH₂)₂-O-(CH₂)₂-, oder
R²-O-(CH₂)₂-O(-CH₂)₂- O-(CH₂)₂- O-(CH₂)₂- steht,
wobei R² für Wasserstoff, Methyl oder Ethyl steht.

9. Verbindung der Formel (IVa-2)

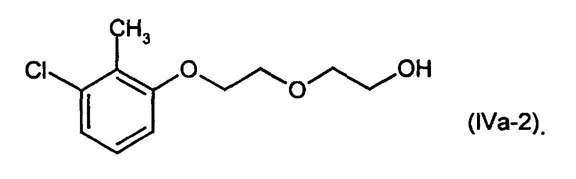

10. Verfahren gemäß mindestens einem der Ansprüche 1 bis 7, **dadurch gekennzeichnet, dass** als Base Kaliumhydroxid verwendet wird.

## Claims

1. Process for preparing compounds of the formula (I) 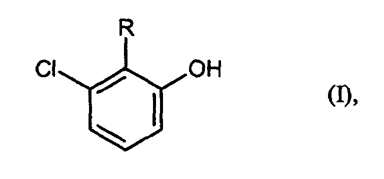 where
R is C₁-C₆-alkyl,
**characterized in that** compounds of the general formula (II) 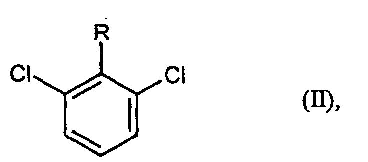 where
R has the above meanings, are reacted
a1) with a base in a high-boiling organic diluent of the general formula (III)
R¹- OH (III),
where
R¹ is R²-O-(CH₂)₂-O-(CH₂)₂-,
R²-O-(CH₂)₂-O(-CH₂)₂-O-(CH₂)₂-,
R²-O-(CH₂)₂-O(-CH₂)₂-O-(CH₂)₂-O-(CH₂)₂- or
-C₆ to C₁₀-alkyl,
where R² is hydrogen, methyl or ethyl,
optionally in the presence of a catalyst, and any water released in the course of the reaction is continuously removed,
or are reacted
a2) with a base in a high-boiling organic diluent of the general formula (III)
R¹- OH (III),
where R¹ has the above meanings,
optionally in the presence of a catalyst, and any water released during the reaction is continuously removed,
and the resulting compounds of the formula (IV) 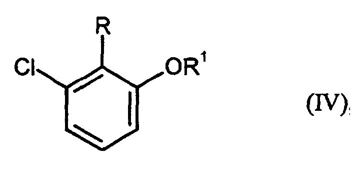 where
R and R¹ have the above meanings,
are treated with relatively highly concentrated acid,
or are reacted
b1) with a base in a primary alcohol having 1 to 3 carbon atoms used as a diluent under pressures of 10 to 40 bar,
or are reacted
b2) with a base in a diluent of the general formula (III)
R¹-OH (III)
in which
R¹ is as defined above,
under pressure,
and the resulting compounds of the formula (IV) 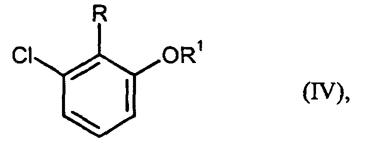 where
R and R¹ have the above meanings,
are treated with relatively highly concentrated acid.

2. Process according to Claim 1, **characterized in that** the diluent used in the process variants a1) and a2) is diethylene glycol.

3. Process according to Claim 1, **characterized in that** the diluent used in the process variants b1) and b2) is methanol.

4. Process according to Claims 1 or 2, **characterized in that** the process variants a1) and a2) are operated at temperatures of from 100°C to 250°C.

5. Process according to any of Claims 1 to 3, **characterized in that** the process variant b1) is operated at temperatures of from 170°C to 250°C.

6. Process according to any of Claims 1 to 3, **characterized in that** the process variant b2) is operated at temperatures of from 100°C to 220°C.

7. Process according to Claim 1, 2 or 4, **characterized in that** the process variant a1) is operated as a one-pot process.

8. Compounds of the formula (IVa) 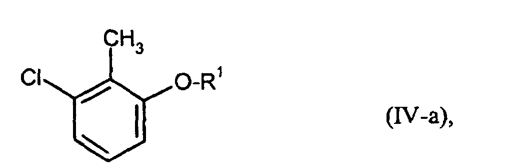 where
R¹ is R²-O-(CH₂)₂-O-(CH₂)₂-,
R²-O-(CH₂)₂-O(-CH₂)₂-O-(CH₂)₂-, or
R²-O-(CH₂)₂-O(-CH₂)₂- O-(CH₂)₂- O-(CH₂)₂-,
where R² is hydrogen, methyl or ethyl.

9. Compound of the formula (IVa-2)

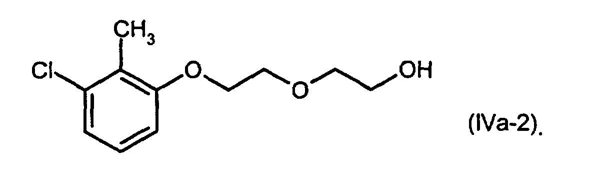

10. Process according to any of Claims 1 to 7, **characterized in that** the base used is potassium hydroxide.

## Revendications

1. Procédé de production de composés de formule (I) 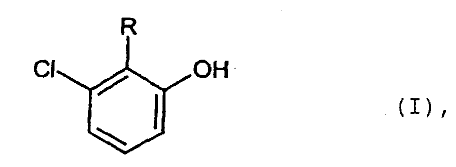 dans laquelle
R est un reste alkyle en C₁ à C₆,
**caractérisé en ce qu'**on fait réagir des composés de formule générale (II) 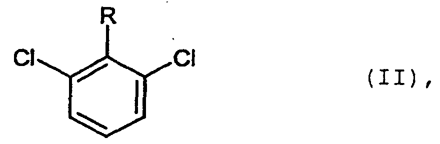 dans laquelle
R a les définitions indiquées ci-dessus,
a1) avec une base dans un diluant organique de haut point d'ébullition de formule générale (III)
R¹- OH (III),
dans laquelle
R¹ représente un groupement
R²-O-(CH₂)₂-O-(CH₂)₂-,
R²-O-(CH₂)₂-O(-CH₂)₂-O-(CH₂)₂-,
R²-O-(CH₂)₂-O(-CH₂)₂-O-(CH₂)₂-O-(CH₂)₂- ou
-(alkyle en C₁ à C₁₀),
où R² est l'hydrogène, un radical méthyle ou éthyle, éventuellement en présence d'un catalyseur, et on élimine en continu l'eau éventuellement libérée au cours de la réaction,
ou bien
a2) avec une base dans un diluant organique de haut point d'ébullition de formule générale (III)
R¹-OH (III),
dans laquelle R¹ a les définitions indiquées ci-dessus,
éventuellement en présence d'un catalyseur, et on élimine en continu l'eau éventuellement libérée au cours de la réaction,
et on traite les composés ainsi produits de formule (IV) 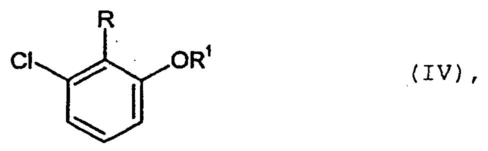 dans laquelle
R et R¹ ont les définitions indiquées ci-dessus,
avec un acide de concentration élevée,
ou bien
b1) avec une base dans un alcool primaire ayant 1 à 3 atomes de carbone utilisé comme diluant, sous des pressions de 10 à 40 bars,
ou bien.
b2) sous pression avec une base dans un diluant de formule générale (III)
**R**^{**1**}**-OH** (III),
dans laquelle
R¹ a les définitions indiquées ci-dessus,
et on traite les composés ainsi produits de formule (IV) 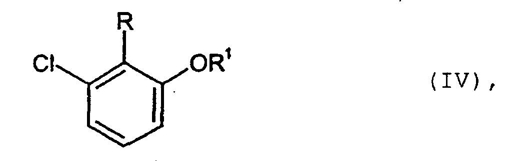
dans laquelle
R et R¹ ont les définitions indiquées ci-dessus, avec un acide de concentration élevée.

2. Procédé suivant la revendication 1, **caractérisé en ce qu'**on utilise comme diluant le diéthylèneglycol dans les variantes opératoires a1) et a2).

3. Procédé suivant la revendication 1, **caractérisé en ce qu'**on utilise le méthanol comme diluant dans les variantes opératoires b1) et b2).

4. Procédé suivant au moins l'une des revendications 1 et 2, **caractérisé en ce que** les variantes opératoires a1) et a2) sont conduites à des températures de 100 à 250°C.

5. Procédé suivant au moins l'une des revendications 1 ou 3, **caractérisé en ce que** la variante opératoire b1) est conduite à des températures de 170 à 250°C.

6. Procédé suivant au moins l'une des revendications 1 ou 3, **caractérisé en ce que** la variante opératoire b2) est conduite à des températures de 100 à 220°C.

7. Procédé suivant au moins l'une des revendications 1, 2 ou 4, **caractérisé en ce que** la variante opératoire a1) est conduite comme procédé en récipient unique.

8. Composés de formule (IVa) 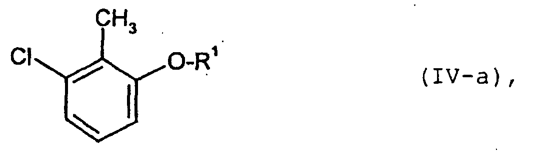 dans laquelle
R¹ représente un groupement
R²-O-(CH₂)₂-O-(CH₂)₂-,
R²-O-(CH₂)₂-O (CH₂)₂-O-(CH₂)₂-, ou
R²-O-(CH₂)₂-O(CH₂)₂-O- (CH₂)₂-O-(CH₂)₂-,
où R² représente l'hydrogène, le radical méthyle ou éthyle.

9. Composé de formule (IVa-2)

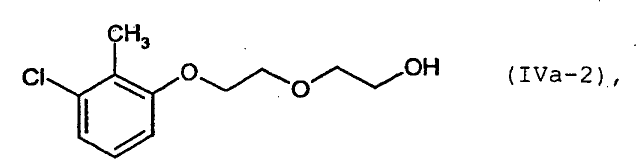

10. Procédé suivant au moins l'une des revendications 1 à 7, **caractérisé en ce que** l'hydroxyde de potassium est utilisé comme base.
